Europäisches Patentamt

European Patent Office

Office européen des brevets

(11) Publication number: **0 383 620**
**A2**

## EUROPEAN PATENT APPLICATION

(21) Application number: 90301700.2

(22) Date of filing: 16.02.90

(51) Int. Cl.⁵: **C12N 15/00, C12N 15/62,**
**A61K 31/785, C12P 21/00**

(30) Priority: 17.02.89 US 312541
07.02.90 US 473845

(43) Date of publication of application:
**22.08.90 Bulletin 90/34**

(84) Designated Contracting States:
**AT BE CH DE DK ES FR GB GR IT LI LU NL SE**

(71) Applicant: **REPLIGEN CORPORATION**
**101 Binney Street**
**Cambridge Massachusetts 02142(US)**

(72) Inventor: **Cook, Kathleen Sue**
**21 Parkman Street, No. 2B**
**Brookline Massachusetts 02146(US)**

(74) Representative: **Perry, Robert Edward et al**
**GILL JENNINGS & EVERY 53-64 Chancery**
**Lane**
**London WC2A 1HN(GB)**

(54) **Process for making genes encoding random polymers of amino acids.**

(57) A method for making a synthetic gene encoding a random polymers of amino-acids but which has predetermined amino acid constituents, comprises the polymerisation of small oligonucleotide duplexes.

The novel synthesis can also be used as part of a method for identifying amino-acid polymers with biological and/or immunological activity.

EP 0 383 620 A2

## PROCESS FOR MAKING GENES ENCODING RANDOM POLYMERS OF AMINO ACIDS

### Background of the Invention

Copolymer 1 (COP-1) is a synthetic polypeptide analog of myelin basic protein (MBP), which is a natural component of the myelin sheath. It has been suggested as a potential therapeutic agent for multiple sclerosis (Eur. J. Immunol. [1971] 1:242; and J. Neurol. Sci. [1977] 31:433). Interest in COP-1 as an immunotherapy for multiple sclerosis stems from observations first made in the 1950's that myelin components such as MBP prevent or arrest experimental autoimmune encephalomyelitis (EAE). EAE is a disease resembling multiple sclerosis that can be induced in susceptible animals.

COP-1 was developed by Drs. Sela, Arnon, and their co-workers at the Weizmann Institute (Rehovot, Israel). It was shown to suppress experimental allergic encephalomyelitis (EAE) (Eur. J. Immunol. [1971] 1:242-248; U.S. Patent No. 3,849,550). More recently, COP-1 was shown to be beneficial for patients with the exacerbating-remitting form of multiple sclerosis (N. Engl. J. Med. [1987] 317:408-414). Patients treated with daily injections of COP-1 had fewer exacerbations and smaller increases in their disability status than the control patients.

COP-1 is a mixture of polypeptides composed of alanine, glutamic acid, lysine, and tyrosine in a molar ratio of approximately 6:2:5:1, respectively. It is synthesized by chemically polymerizing the four amino acids forming products with average molecular weights of 23,000 daltons. Although the resulting polypeptides are comprised of the same amino acid components, they differ with respect to their amino acid sequences. In fact, there are $10^{100}$ possible ways to assemble a 23,000 dalton polypeptide composed of alanine, glutamic acid, lysine and tyrosine in the designated ratios. Purification of one or even a small number of distinct COP-1 polypeptides from chemically-synthesized COP-1 is not possible.

Studies evaluating COP-1's efficacy have been hindered somewhat by inconsistent batches of COP-1. Also, it is not known which of the amino acid polymer(s) is responsible for the biological activity of COP-1. Other random sequence amino acid copolymers related to COP-1 have been chemically synthesized and tested for the ability to suppress experimental allergic encephalomyelitis (Eur. J. Immunol. [1973] 3:273:; Immunochemistry [1976] 13:333). Biological activity was observed in EAE assays using COP-1 related polymers in which one of the following changes occurs: tyrosine is replaced by tryptophan; or glutamic acid is replaced by aspartic acid; or tyrosine is excluded.

We have developed procedures for synthesizing genes encoding polypeptides composed of specific amino acids, but having random amino acid sequences. The amino acid composition of the polypeptides is dictated by the set of codons incorporated in the synthetic genes. Likewise, the size of the polypeptides is controlled by synthesizing genes of specific lengths.

### Brief Summary of the Invention

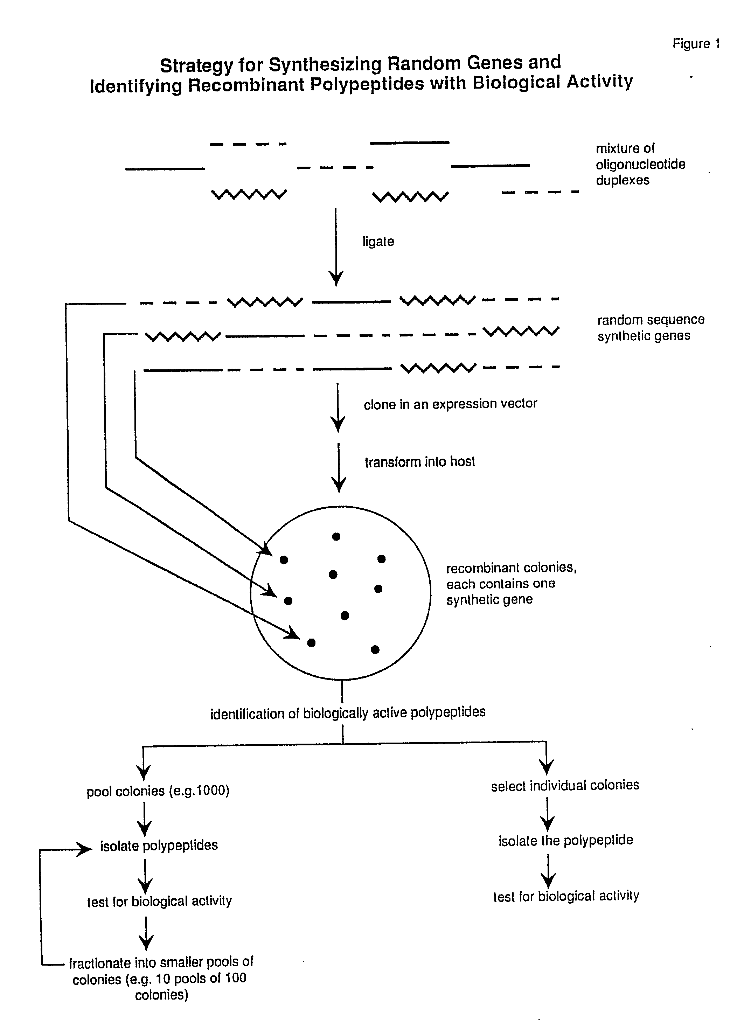

The subject invention concerns a method for synthesizing genes which encode random polymers of amino acids. A further aspect of the invention is the identification of certain polypeptides which are expressed by the synthetic genes and which have high levels of biological activity. The general methodology of the subject invention is outlined in Figure 1.

A critical step in the novel process of the subject invention is the polymerization of small oligonucleotide duplexes. Preferably, the oligonucleotide duplexes consist of a multiple of three nucleotides. The length of the synthetic genes can be controlled through the use of adaptors specific for the 5' and 3' ends of the oligonucleotides. Further, the composition of the resultant polypeptides can be varied, with respect to the relative proportions of the amino acid constituents, by varying the proportions of input oligonucleotide duplexes.

The synthesis of polypeptides similar to COP-1 exemplifies the procedures of the subject invention. The initial step in the procedure is the synthesis of genes which code for polypeptides consisting of predetermined amino acid constituents. These genes are then cloned in an expression vector and introduced into E. coli such that each recombinant bacterial colony contains one COP-1 gene. To generate a mixture of COP-1 polypeptides (analogous to the chemically synthesized product) we produce COP-1 polypeptides from a pool of recombinant bacterial colonies containing COP-1 gene sequences, e.g., 1000 colonies.

The efficacy of the pool of recombinant COP-1 polypeptides is tested in experimental allergic encephalomyelitis (EAE) assays. If effective, the pool of colonies exhibiting activity is further subdivided (e.g., pools of 100 colonies), and the polypeptides from these smaller pools are tested. By sequentially fractionating and selecting the most active pools, we identify individual recombinant COP-1 polypeptides or small groups of polypeptides with biological activity in EAE assays equal to or higher than chemically synthesized COP-1. The opportunity to characterize homogeneous, individual COP-1 polypeptides is unique to this approach.

The subject invention also concerns the synthetic genes and the polypeptides produced by the methods disclosed herein. Advantageously, the procedures of the subject invention can be used to produce polypeptides which may be useful in preventing, arresting, or controlling demyelinating disorders such as multiple sclerosis. A preferred copolymer according to the subject invention consists substantially of alanine, lysine, and either glutamic or aspartic acid. Polymers of any length or molecular weight can be synthesized using the procedures of the subject invention. Another preferred copolymer further includes either tyrosine or tryptophan. More specifically, a preferred copolymer may consist of alanine, lysine, glutamic acid, and tyrosine, and have a molecular weight between about 5,000 and 50,000 daltons. Further, the method of the subject invention can also be used to make fusion proteins.

Brief Description of the Drawings

Figure 1 depicts the general methodology for synthesizing random genes and identifying polypeptides with biological activity.

Figure 2 shows one specific strategy for synthesizing genes encoding random-sequence polypeptides.

Figure 3 shows all possible 3-amino acid combinations and their percent occurrence in COP-1.

Figure 4 shows 9-nucleotide duplexes and adaptors for COP-1 gene synthesis.

Figure 5 shows the construction and cloning of synthetic COP-1 genes.

Figure 6 provides the sequence analysis of one synthetic gene revealing proper junctions between duplexes.

Figure 7 is a Western blot showing the fusion protein produced by four different clones.

Figure 8 shows variations of random-gene synthesis using small duplexes.

Figure 9 shows synthesis of single-stranded DNA encoding random sequence amino acid polymers.

Figure 10 shows a phosphoramidite trinucleotide for random gene synthesis using a DNA synthesizer.

Figure 11 shows the DNA and amino acid sequences of rCOP-1-77.

Figure 12 shows the DNA and amino acids sequences of rCOP-1-19.

Figure 13 shows the average EAE scores of disease-induced guinea pigs which were untreated or treated with myelin basic protein, rCOP-1-19, or rCOP-1-77.

Detailed Description of the Invention

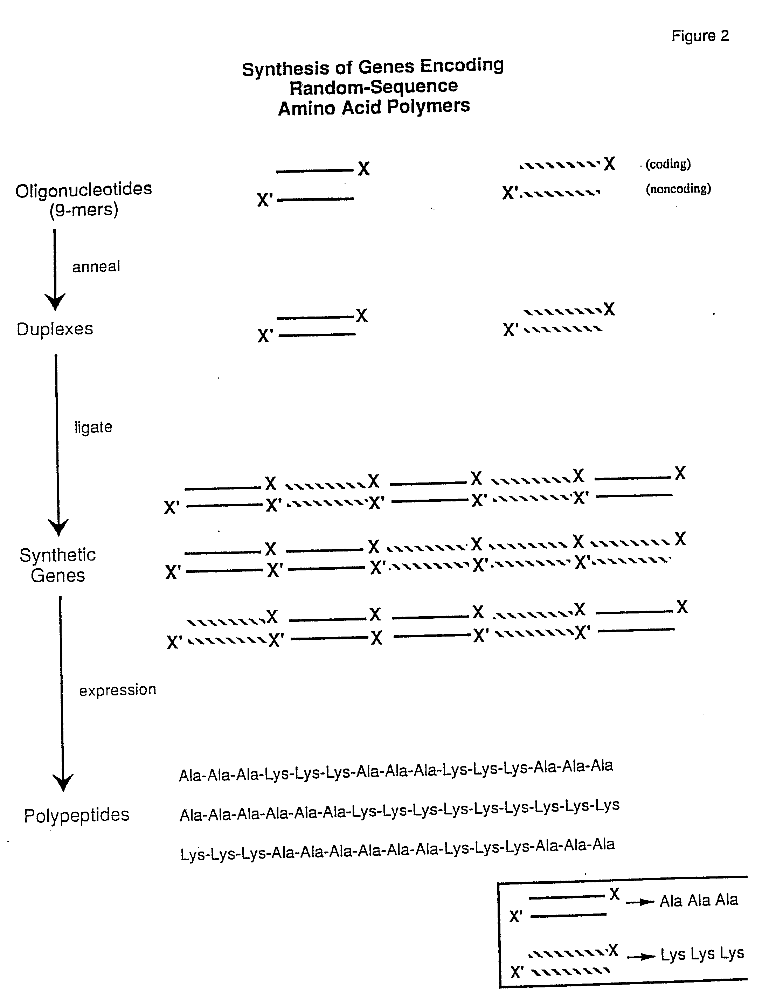

One strategy for synthesizing genes encoding random-sequence polypeptides is outlined in Figure 2. The example illustrates the synthesis of genes composed of two DNA duplexes. Oligonucleotides comprising the duplexes are synthesized and annealed. Each DNA duplex has the same "sticky ends" represented by X and X′ in Figure 2. The duplexes are mixed together, sticky ends align, and the ends are joined in an enzymatic reaction producing long segments of DNA (genes). Since the sticky ends on each duplex are the same, the duplexes can align and ligate in any order. Thus, a series of genes composed of the same duplexes but in varying orders are produced. The polypeptides encoded by these genes will have similar amino acid compositions but different sequences.

The synthesis of random-sequence genes requires significant modifications of the procedures used previously to construct genes encoding specific proteins. First, random sequence genes are synthesized by assembling small oligonucleotide duplexes because more sequence variation is produced by mixing the order of small rather than large duplexes. In contrast, long oligomers of 30 to 60 nucleotides are employed to synthesize a gene encoding a defined amino acid sequence. Secondly, to construct random-sequence genes, the sticky ends on each duplex must be identical so that the duplexes can be joined together in any order. For genes corresponding to defined amino acid sequences, the duplexes must ligate in a fixed order.

To achieve this ordering, the sticky ends at each junction must be unique. Thus, the process of the subject invention is unique in that random-sequence genes are synthesized using oligonucleotide duplexes encoding small segments of amino acids, and the sticky ends on each duplex are the same.

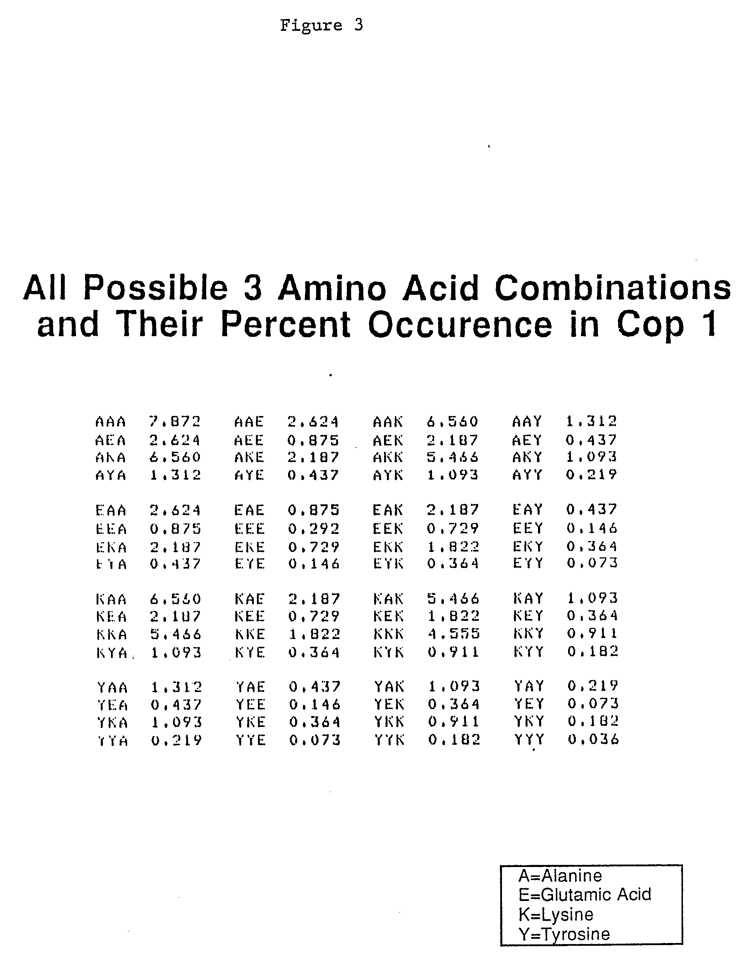

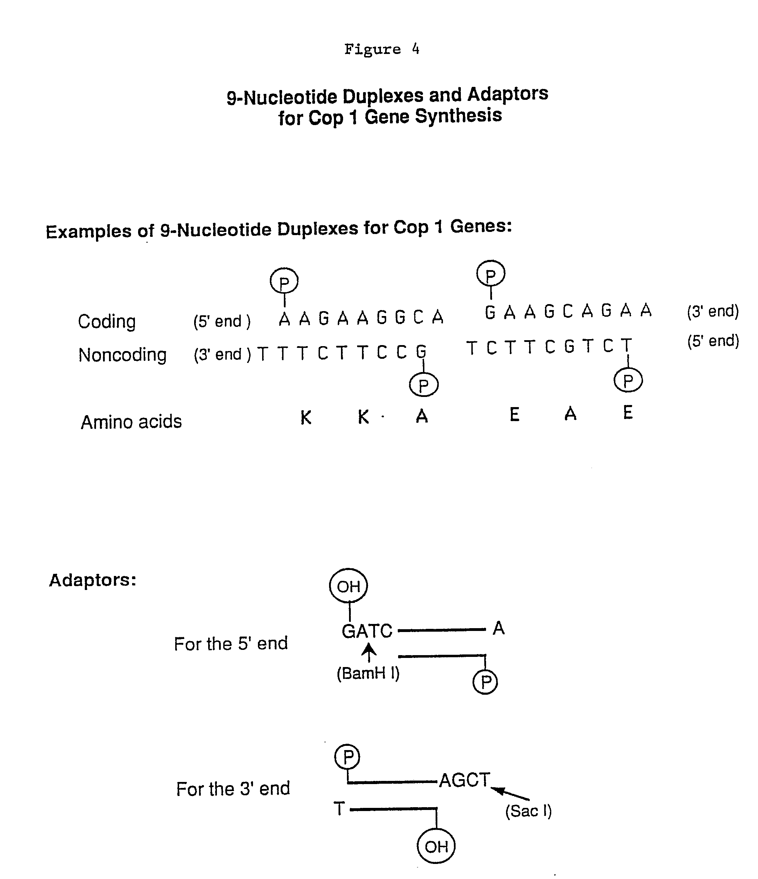

Our procedure for synthesizing genes encoding recombinant COP-1 polypeptides entails using oligonucleotide duplexes encoding segments of 3 amino acids. All possible permutations of the 3 amino acid segments comprised of the four COP-1 amino acids and their percent occurrences in COP-1 are shown in Figure 3. To make recombinant COP-1 genes we have synthesized oligonucleotides corresponding to the coding and noncoding strands for some of the 3 amino acid segments (Figure 4). The oligonucleotides are phosphorylated at the 5' ends. Complementary pairs of oligonucleotides are annealed, forming duplexes with the 3' nucleotide extending on each strand: adenosine on the coding strand, and thymidine on the noncoding strand. Adenosine and thymidine base pair with one another thus ensuring that the duplexes are joined directionally, that is, coding strands to other coding strands. Since the duplexes have the same nucleotide extensions, they can align in any order. When duplexes corresponding to all sixty-four 3-amino acid blocks are mixed and ligated, COP-1 genes with all possible sequences are produced.

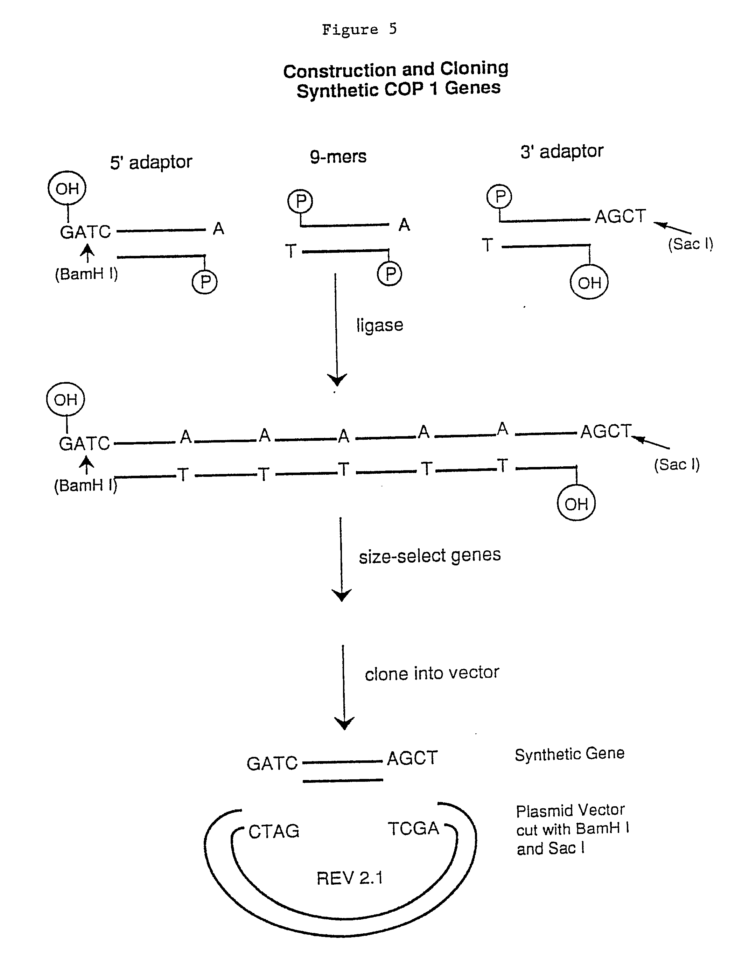

To control the length of the synthetic genes, we have included adaptors specific for the 5' and 3' ends. One strand of each duplex adaptor is not phosphorylated (see Figure 4). As a result, ligation products terminate at the hydroxylated ends. By varying the ratio of the adaptor duplexes in the reaction, we can control the length of the synthetic genes. The adaptors serve the second function of adding specific extensions required to directionally clone the ligation products into the vector (Figure 5). The relative proportions of amino acid constituents in the random polymers can also be modulated by mixing the oligonucleotide duplexes in different ratios. For example, duplexes can be added to ligations in proportions dictated by the percent occurrence of the corresponding amino acid segments in COP-1 (Figure 3).

The number of different duplexes incorporated determines the sequence complexity of the synthetic genes. For example, COP-1 genes can be constructed using fewer than 64 duplexes but not all sequence combinations will occur. The amount of sequence complexity required will depend on the application of the polypeptides.

One complication arises in producing completely random COP-1 amino acid sequences. For duplexes to have the same extensions, the 3' nucleotide on the coding strands must be the same. Codons for alanine, glutamic acid, and lysine can end with adenosine; however, for tyrosine, the last nucleotide is either cytidine or uridine. Thus, duplexes encoding three amino acid segments ending in tyrosine (fourth column on Figure 3) will have different extensions than the other duplexes. This limitation can be overcome by making a second noncoding strand for each duplex with an extension of guanosine or adenosine. Because this solution requires significantly more DNA synthesis, we have elected to exclude duplexes corresponding to three amino acid segments ending in tyrosine.

Once the synthetic genes are made, they are cloned into a suitable expression vector and transferred to a host capable of expressing the polypeptides. The host may be bacterial or eukaryotic cells. With bacteria, cells are grown under conditions that permit formation of recombinant colonies. Each colony will contain and express one synthetic gene. The polypeptides expressed by culture from specific colonies are isolated and tested for the relevant biological or immunological activity. For example, COP-1 polypeptides are tested for the ability to suppress EAE.

To screen a large number of polypeptides for activity, it may be advantageous to pool the polypeptides before testing. Pools of polypeptides can be generated by either combining colonies and isolating the polypeptides produced by the mixed culture or by culturing individual colonies, isolating the polypeptides from each culture, and then combining the purified polypeptides. By testing pools of polypeptides, it is possible to more quickly determine which of the colonies express biologically or immunologically active polypeptides. For example, if polypeptides from 100 colonies do not exhibit activity, these colonies can be eliminated from further investigation. If, on the other hand, the mixture of polypeptides does exhibit the desired activity, then sequential subsets of the pooled polypeptides can be tested until the active polypeptides, or mixture of polypeptides, is identified.

Tests for some biological properties may be performed directly on the colonies, thus eliminating the need for isolating polypeptides. For example, reactivity of polypeptides with antisera can be assessed in colonies grown and lysed on nitrocellulose filters.

## Materials and Methods

4

Synthesis and Phosphorylation of Oligonucleotides. The coding and noncoding oligonucleotides for each duplex are synthesized by the phosphite triester method with an Applied Biosystems Model 380 DNA synthesizer. The 5′ ends of oligonucleotides are phosphorylated on the DNA synthesizer using (2-[2-(4,4′-dimethoxytrityloxy)ethylsulfonyl)ethyl-(2-cyanoethyl)N,N-diisopropyl) phosphoramidite from Glen Research, Herndon, VA.

Purification of Oligonucleotides. The phosphorylated form of the oligonucleotide is separated from the crude mixture by electrophoresis through a 20% acrylamide gel containing 7 M urea. Oligomers are eluted from excised gel slices and desalted on Sep-Pak C-18 cartridges. Separation of the 5′ phosphorylated oligomer from hydroxylated forms is critical because hydroxylated oligomer causes the ligation reactions to terminate prematurely, yielding very small products.

Annealing and Ligation of Oligonucleotides. Mixtures containing 1 nmol each of a coding and complementary noncoding strand, 100 mM Tris-HCl, pH 7.6, and 0.1 mM ethylenediaminetetraacetic acid (EDTA) in 50 microliters are heated to 80°C in a 600 ml water bath. The reactions are allowed to cool slowly (2 hours) to room temperature. The temperature is decreased to 4°C over one hour by adding ice to the water bath. For ligation, equal aliquots of the annealed duplexes are mixed and the solution is adjusted to contain 10 mM MgCl$_2$, 1 mM adenosine triphosphate (ATP), 1 mM DTT, and 15% polyethylene glycol. The final concentration of Tris-HCl is 66 mM (from the annealing reactions). The total concentration of 9-mer duplexes is 10 pmol/microliter. Annealed adaptors are included in the reactions at a concentration which will produce ligation products of the correct size and with termini that are compatible with sites in the cloning vector. To obtain a maximum yield of synthetic genes within the correct size range, a series of ligation reactions are set up with increasing ratios of adaptor duplex:9-mer duplex (e.g., 1:50, 1:150, 1:300). The reactions yielding products in the desired size range are pursued. We have determined that adding 1 pmol of each adaptor duplex to 50-300 pmol of 9-mer duplexes yields products of 400-600 base pairs. Ligation reactions are in 75 microliters with 600 units of T4 DNA ligase (New England Biolabs, Beverly, MA). The reaction proceeds at 16°C for 16-20 hours. After ligation, polyethylene glycol is removed by extraction with 3 volumes of chloroform. The products are concentrated by ethanol precipitation and resuspended in 10 microliters.

Size Selection of Ligation Products. The concentrated reaction products are electrophoresed on 4% NuSieve GTG agarose (FMC Bioproducts, Rockford, ME) gels. Products are detected by staining with ethidium bromide, and the region corresponding to the desired size range (400-600 nucleotides) is excised. Synthetic genes of 400-600 nucleotides encode polypeptides of 15,000-23,000 daltons. We have selected genes of approximately this size because COP-1 polypeptides within this range were previously tested in chemical trials. The agarose plug containing the synthetic DNA is stored at -20°C.

## Preparation of Expression Vector

The pREV 2.1 plasmid can be constructed from a plasmid pBG1. Plasmid pBG1 can be isolated from its E. coli host by well known procedures, e.g., using cleared lysate-isopycnic density gradient procedures, and the like. Plasmid pBG1 was deposited in the E. coli host MS371 with the Northern Regional Research Laboratory (NRRL, U.S. Department of Agriculture, Peoria, Illinois, U.S.A) on November 1, 1984, and was assigned the accession number NRRL B-15904. pREV 2.1 was constructed from plasmid expression vector pREV 2.2. Like pBG1, pREV2.2 expresses inserted genes behind the E. coli promoter. The differences between pBG1 and pREV2.2 are the following:

1. pREV2.2 lacks a functional replication of plasmid (rop) protein.

2. pREV2.2 has the trpA transcription terminator inserted into the AatII site. This sequence insures transcription termination of over-expressed genes.

3. pREV2.2 has genes to provide resistance to ampicillin and chloramphenicol, whereas pBG1 provides resistance only to ampicillin.

4. pREV2.2 contains a sequence encoding sites for several restriction endonucleases.

The following procedures were used to make each of the four changes listed above:

1a. 5 μg of plasmid pBG1 was restricted with NdeI, which gives two fragments of approximately 2160 and 3440 base pairs.

1b. 0.1 μg of DNA from the digestion mixture, after inactivation of the NdeI, was treated with T4 DNA ligase under conditions that favor intramolecular ligation (200 μl reaction volume using standard T4 ligase reaction conditions [New England Biolabs, Beverly, MA]). Intramolecular ligation of the 3440 base pair fragment gave an ampicillin resistant plasmid. The ligation mixture was transformed into the recipient strain E. coli JM103 (available from New England Biolabs) and ampicillin resistant clones were selected by standard procedures.

1c. The product plasmid, pBG1 N, where the 2160 base pair NdeI fragment is deleted from pBG1, was selected by preparing plasmid from ampicillin resistant clones and determining the restriction digestion patterns with NdeI and SalI (product fragments approximately 1790 and 1650). This deletion inactivates the rop gene that controls plasmid replication.

2a. 5 μg of pBG1 N was then digested with EcoRI and BclI and the larger fragment, approximately 2455 base pairs, was isolated.

2b. A synthetic double stranded fragment was prepared by the procedure of Itakura et al. (Itakura, K., J.J. Rossi, and R.B. Wallace [1984] Ann. Rev. Biochem. 53:323-356, and references therein) with the following structure:

$$5'\quad \text{GATCAAGCTTCTGCAGTCGACGCATG}$$

$$3'\quad \text{TTCGAAGACGTCAGCTGCGTACGCCT}$$

$$\text{AGGCCATGGGCCCTCGAGCTTAA}\quad 5'$$

$$\text{CGGATCCGGTACCCGGGAGCTCG}\quad 3'$$

This fragment has BclI and EcoRI sticky ends and contains recognition sequences for several restriction endonucleases.

2c. 0.1 μg of the 2455 base pair EcoRI-BclI fragment and 0.01 μg of the synthetic fragment were joined with T4 DNA ligase and competent cells of strain JM103 were transformed. Cells harboring the recombinant plasmid, where the synthetic fragment was inserted into pBG1 N between the BclI and EcoRI sites, were selected by digestion of the plasmid with HpaI and EcoRI. The diagnostic fragment sizes are approximately 2355 and 200 base pairs. This plasmid is called pREV1.

2d. 5 μg of pREV1 were digested with AatII, which cleaves uniquely.

2e. The following double-stranded fragment was synthesized:

5′ CGGTACCAGCCCGCCTAATGAGCGGGCTTTTTTTTTGACGT 3′
3′ TGCAGCCATGGTCGGGCGGATTACTCGCCCGAAAAAAAAAC 5′

This fragment has AatII sticky ends and contains the trpA transcription termination sequence.

2f. 0.1 μg of AatII digested pREV1 was ligated with 0.01 μg of the synthetic fragment in a volume of 20 μl using T4 DNA ligase.

2g. Cells of strain JM103, made competent, were transformed and ampicillin resistant clones selected.

2h. Using a KpnI, EcoRI double restriction digest of plasmid isolated from selected colonies, a cell containing the correct construction was isolated. The sizes of the KpnI, EcoRI generated fragments are approximately 2475 and 80 base pairs. This plasmid is called pREV1TT and contains the trpA transcription terminator.

3a. 5 μg of PREV1TT, prepared as disclosed above (by standard methods) was cleaved with NdeI and XmnI and the approximately 850 base pair fragment was isolated.

3b. 5 μg of plasmid pBR325 (BRL, Gaithersburg, MD), which contains the genes conferring resistance to chloramphenicol as well as to ampicillin and tetracycline, was cleaved with BclI and the ends blunted with Klenow polymerase and dexoynucleotides. After inactivating the enzyme, the mixture was treated with NdeI and the approximately 3185 base pair fragment was isolated. This fragment contains the genes for chloramphenicol and ampicillin resistance and the origin of replication.

3c. 0.1 μg of the NdeI-XmnI fragment from pREV1TT and the NdeI-BclI fragment from pBR325 were ligated in 20 μl with T4 DNA ligase and the mixture used to transform competent cells of strain JM103. Cells resistant to both ampicillin and chloramphenicol were selected.

3d. Using an EcoRI and NdeI double digest of plasmid from selected clones, a plasmid was selected giving fragment sizes of approximately 2480, 1145, and 410 base pairs. This is called plasmid pREV1TT/chl and has genes for resistance to both ampicillin and chloramphenicol.

4a. The following double-stranded fragment was synthesized:

| MluI | EcoRV | ClaI BamHI |
|---|---|---|

5' CGAACGCGTGGCCGATATCATCGATGG

3' GCTTGCGCACCGGCTATAGTAGCTACC

| SalI HindIII | SmaI |
|---|---|

ATCCGTCGACAAGCTTCCCGGGAGCT        3'

TAGGCAGCTGTTCGAAGGGCCC        5'

This fragment, with a blunt end and an SstI sticky end, contains recognition sequences for several restriction enzyme sites.

4b. 5 $\mu$g of pREV1TT/chl was cleaved with NruI (which cleaves about 20 nucleotides from the Bc1I site) and SstI (which cleaves within the multiple cloning site). The larger fragment, approximately 3990 base pairs, was isolated from an agarose gel.

4c. 0.1 $\mu$g of the NruI-SstI fragment from pREV1TT/chl and 0.01 $\mu$g of the synthetic fragment were treated with T4 DNA ligase in a volume of 20 $\mu$l.

4d. This mixture was transformed into strain JM103 and ampicillin resistant clones were selected.

4e. Plasmid was purified from several clones and screened by digestion with MluI or ClaI. Recombinant clones with the new multiple cloning site will give one fragment when digested with either of these enzymes, because each cleaves the plasmid once.

4f. The sequence of the multiple cloning site was verified. This was done by restricting the plasmid with HpaI and PvuII and isolating the 1395 base pair fragment, cloning it into the SmaI site of mp18 and sequencing it by dideoxynucleotide sequencing using standard methods.

4g. This plasmid is called pREV2.2.

Plasmid pREV 2.2 can be isolated from its E. coli host by well known procedures. This plasmid was deposited in the E. coli host JM103 with the Northern Regional Research Laboratory (NRRL, U.S. Department of Agriculture, Peoria, Illinois, USA) on July 20, 1986 and was assigned the accession number NRRL B-18091.

Plasmid pREV 2.1 was constructed using plasmid pREV 2.2 and a synthetic oligonucleotide. An example of how to construct pREV 2.1 is as follows:

1. Plasmid pREV 2.2 is cleaved with restriction enzymes NruI and BamHI and the 4 Kb fragment is isolated from an agarose gel.

2. The following double-strand oligonucleotide is synthesized:

5' CGAACGCGTGGTCCGATATCATCGATG        3'

3' GCTTGCGCACCAGGCTATAGTAGCTACCTAG        5'

3. The fragments from 1 and 2 are ligated in 20 ul using T4 DNA ligase, transformed into competent E. coli cells and chloramphenicol resistant colonies are isolated.

4. Plasmid clones are identified that contain the oligonucleotide from 2, spanning the region from the NruI site to the BamHI site and recreating these two restriction sites. This plasmid is termed pREV 2.1.

Cloning the Ligation Products into an Expression Vector. The expression vector is digested to yield extensions that are compatible with the adaptors at the termini of the synthetic genes. The agarose plug containing the size-selected synthetic genes is melted at 65-70° C. and transferred to 37° C. Nine microliters of melted agarose containing the genes is mixed with one microliter of digested vector (20 ng). The mixture is adjusted to contain 66 mM Tris-HCl, pH 7.5, 5 mM MgCl₂, 5 mM DTT, and 1 mM ATP and is diluted to 20 microliters. T4 DNA ligase (400 units) is added and the reaction is incubated overnight at 16° C. Competent cells are transformed with the ligation mixtures and recombinants are identified by selection on plates containing appropriate drugs.

Following are examples which illustrate procedures, including the best mode, for practicing the invention. These examples should not be construed as limiting. All percentages are by weight and all solvent mixture proportions are by volume unless otherwise noted.

Example 1 - Strategy for Synthesis of Random Sequence Genes

Model studies using several oligonucleotide duplexes were performed to assess this method for synthesizing genes encoding polypeptides of predetermined amino acid composition but random sequences. The synthetic genes were analyzed with respect to size, ligation junctions, composition, sequence, and levels of expression.

Size. Synthetic genes within broad size ranges are produced by varying the ratio of adaptors to 9-mer duplexes. We are able to select genes within more limited size ranges by resolving the ligation products on agarose gels and excising gel slices containing products of a certain length. Using these procedures, genes in the following three size ranges have been isolated: 75-150, 280-320, and 400-600 nucleotides.

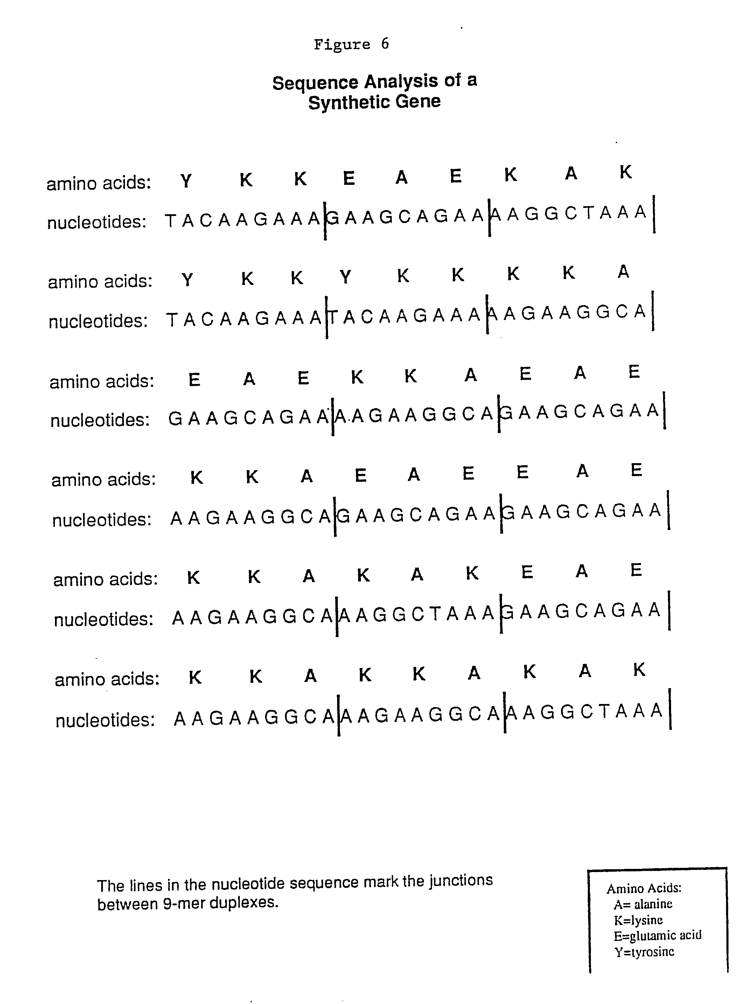

Ligation junctions. The synthetic genes have been sequenced to demonstrate that the 9-mer duplexes are joined end to end and without insertion or deletion of any nucleotides. Correct junctions are necessary for maintaining the reading frame and thus producing genes that encode polypeptides of the expected amino acid composition. Sequence analysis revealed that the junctions between the duplexes are correct (Figure 6).

Composition. To control the amino acid composition of the encoded polypeptides, the synthetic genes must contain the duplexes added to the ligation reactions. The results from three gene synthesis experiments demonstrated that the synthesized genes are composed of the duplexes included in the input steps of the synthesis.

Sequence. Since each duplex can ligate to any other, the order of the 9-mer duplexes in the synthetic genes should be random. This randomness is demonstrated in the Example shown in Figure 6. The synthetic gene shown in this example is composed of duplexes encoding the following amino acid segments: KKA, EAE, KAK and YKK.

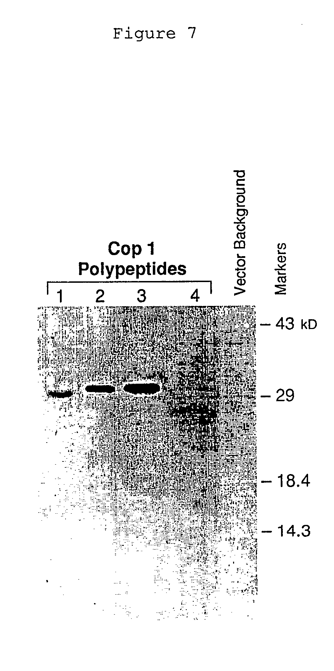

Expression. To measure expression levels, synthetic genes are cloned into vectors such that the polypeptides are expressed either as fusions with heterologous vector-derived peptide sequences, or as nonfusion polypeptides. The levels of expression of the fusion products can be readily measured by Western blot analysis using antisera directed against the vector-derived portion of the fusion protein. Figure 7 demonstrates the expression of four COP-1-containing fusion polypeptides.

Example 2 - Variations for Synthesizing Random Sequence Genes with Small DNA Duplexes

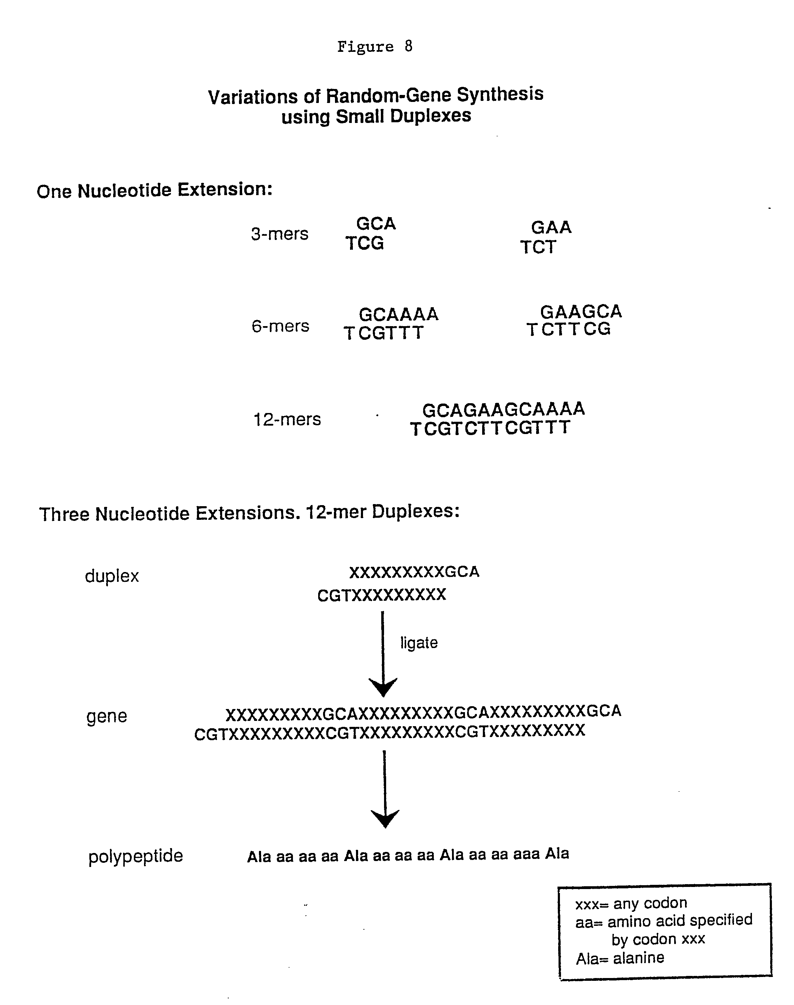

DNA duplexes of other lengths can be used in an approach similar to that described for duplexes of 9 nucleotides. Since three nucleotides code for one amino acid, strands of 3, 6, 12, 15, or 18 nucleotides can be annealed forming duplexes that code for small blocks of amino acids (see Figure 8). More sequence variation occurs by mixing small rather than large duplexes.

In addition, terminal extensions of more than one nucleotide can be employed. Figure 8 shows an example using duplexes of 12 nucleotides and extensions of 3 nucleotides. XXX represents any codon; the codons in the duplexes are varied to produce polypeptides of the desired amino acid composition. This approach restricts the polypeptide sequences since the amino acid encoded by the duplex junctions -- alanine (Ala) in this example -- is repeated every fourth amino acid. Also, in another variation of the subject invention, extensions of 5′ nucleotides can be employed instead of the 3′ extensions illustrated throughout this report.

Example 3 - Synthesis of Single-Stranded Random Sequence Genes

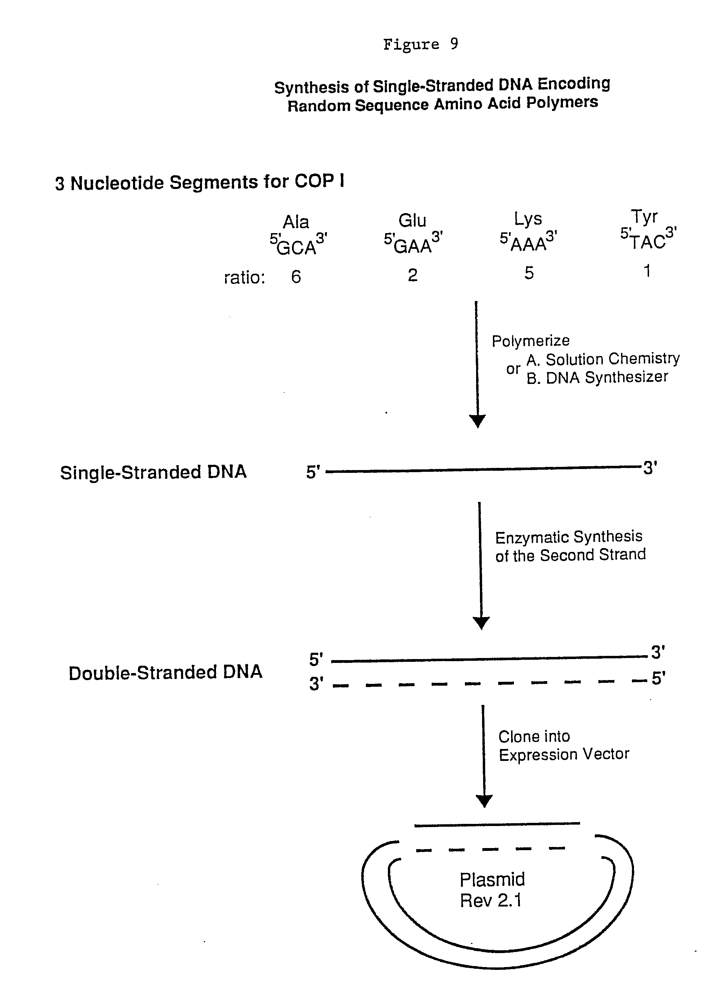

Gene synthesis using DNA duplexes results in double stranded genes that are ready for cloning into an expression vector. An alternative strategy entails producing single-stranded, random sequence genes. The application of this method to COP-1 is illustrated in Figure 9. Three-nucleotide oligomers corresponding to codons for each of the amino acids in COP-1 are synthesized, mixed in appropriate ratios, and chemically polymerized in solution to produce long single-stranded COP-1 genes. The complementary strand of DNA is made enzymatically using reverse transcriptase or DNA polymerase. The double-stranded DNA is prepared for cloning by digestion and repairing the ends of the molecules.

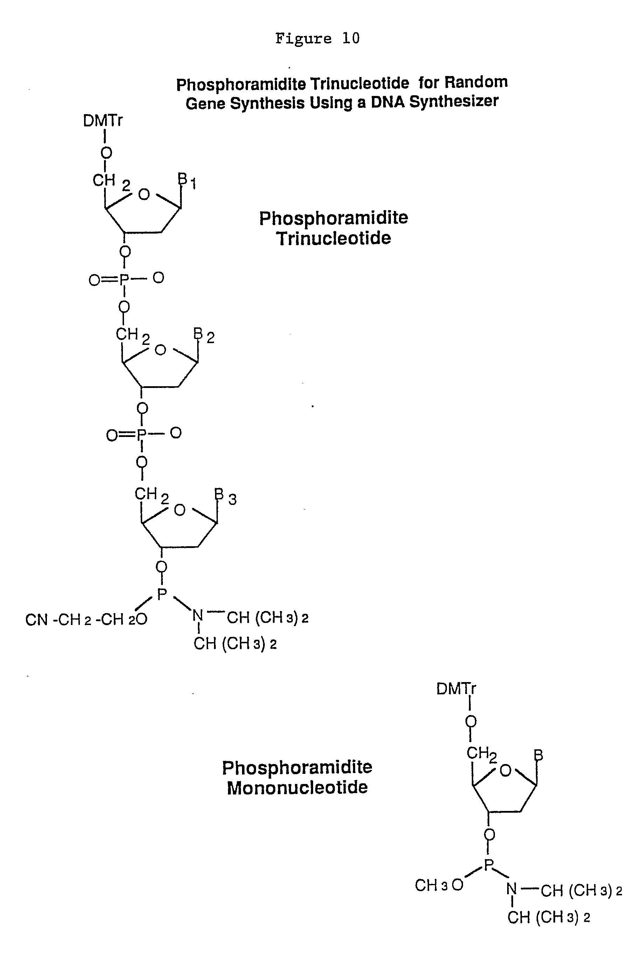

Single-stranded, random sequence genes could also be made by performing the polymerization step on a DNA synthesizer. Typically, synthetic DNA is assembled one nucleotide at a time using phosphoramidite nucleotide precursors. We developed a strategy for synthesizing single-stranded DNA in three nucleotide segments ("codons") by using phosphoramidite trinucleotides (Figure 10). The use of 3-nucleotide building

8

blocks instead of single nucleotides is necessary to ensure that only specific codons, those corresponding to the phosphoramidite trinucleotides, would occur in the synthetic genes. To test this strategy, we commissioned the custom synthesis of a phosphoramidite trinucleotide. We observed polymerization of the trinucleotide on the DNA synthesizer.

Example 4 - Expression of Random Sequence Genes in Vector Producing Fusion Proteins

Synthetic random sequence genes can be cloned into gene fusion vectors so that the expressed polypeptides are comprised of a vector-derived polypeptide linked to the random sequence polypeptide. The application of this method to COP-1 is described here. Synthetic COP-1 genes are cloned into the expression vector denoted pREV 2.1 within the polylinker site. Upon expression from pRev 2.1, a polypeptide is synthesized comprising the amino-terminal portion (approximately 25 to approximately 45 amino acids) of the bacterial protein linked by a peptide bond to the COP-1 polypeptide.

We have tested twelve different COP-1 genes for expression in pRev 2.1. Fusion polypeptides were found to be expressed from ten of the twelve constructs. Figure 7 is a Western blot demonstrating the fusion proteins produced from four different clones, as detected by binding of antisera specific for the bacterial portion of the fusion protein. Detection of fusion proteins in the expected size range using antisera specific for the vector-derived portion is dependent on the presence of a COP-1 gene sequence since the bacterial peptide alone is much smaller.

Clones 1, 2, and 3 each produce fusion polypeptides comprised of 34 amino acids of the vector-derived protein (approximately 3,900 daltons) and approximately 130-200 amino acids encoded by the random sequence genes (15,000-23,000 daltons). Based on migration through SDS gels, the molecular weights of the fusion polypeptides are in the correct range. Clone 4 produces lower levels of several smaller polypeptides which may be generated upon degradation of the largest species.

The amino acids which are at the junction of the vector-derived and COP-1 polypeptides are encoded by the 5' oligonucleotide adaptor duplex. The adaptor duplex can be designed to encode a methionine residue between the bacterial protein and the COP-1 sequences. In this case, the COP-1 polypeptides can be released from the fusion protein by treatment with cyanogen bromide which cleaves on the carboxyl terminal side of methionine residues. Both forms of COP-1 (the fusions and the free polypeptides) are tested for biological activity.

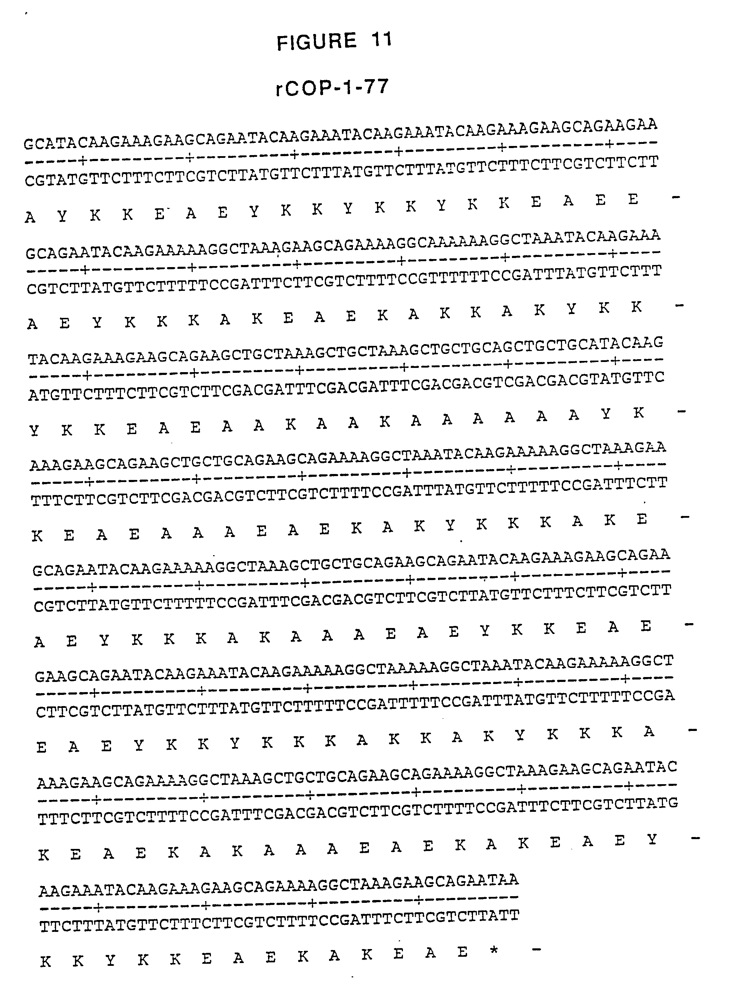

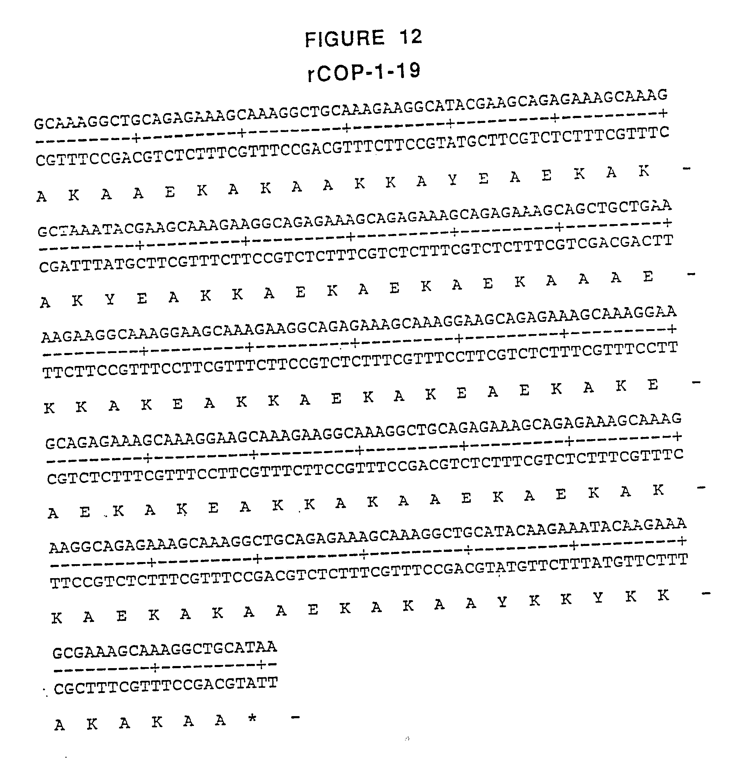

In addition to pRev 2.1, other fusion vectors can be employed to express COP-1 fusion polypeptides, and other strategies can be employed to release the COP-1 polypeptides from the bacterial proteins. For example, to improve the expression of rCOP-1 polypeptides in E. coli, genes coding for rCOP-1-77 and rCOP-1-19, were subcloned from pREV 2.1 to pBG3-2ΔN, a plasmid used to express Protein A. pBG3-2ΔN has been deposited as described in U.S. Patent No. 4,691,009. The deposit was made on November 20, 1984 and given the accession number of NRRL B-15910. The rCOP-1 genes were isolated from the pREV 2.1 recombinant plasmids by digestion with Nco1 and EcoR1. The Nco1 site occurs in the 5' linker employed in cloning the rCOP-1 genes and the EcoR1 site is in pREV 2.1 downstream of the rCOP-1 gene. After digestion with the restriction enzymes, the ends of the rCOP-1 genes are blunted with deoxyribonucleotides and Klenow fragment. DNA fragments containing the rCOP-1 genes are isolated from agarose gels. pBG3-2ΔN is digested wtih Nhe1, treated with phosphatase and the ends of the DNA are blunted with deoxyribonucleotides and Klenow fragment. After ligation and plating, pBG3-2ΔN recombinants bearing rCOP-1 genes in the correct orientation are identified by DNA sequence analysis. The resulting plasmids encode fusion proteins consisting of β-glucuronidase, Protein A, and rCOP-1 sequences. A methionine residue occurs between the Protein A and rCOP-1 sequences, originating from the 5' linker sequence, in order that the COP-1 polypeptide may be cleaved from the fusion protein. The nucleotide and amino acid sequences for rCOP-1-77 and rCOP-1-19 are shown in Figures 11 and 12, respectively. rCOP-1-19 contains oligonucleotide duplexes encoding the following amino acid segments: YKK, AAE, KAK, EKA, KKA, YEA, AKA, KEA, and KAA. rCOP-1-77 contains oligonucleotide duplexes encoding the following amino acid segments: YKK, EAE, KAK AAK, and AAA. The N-terminal alanine residue in each sequence is left behind following CNBr cleavage of the fusion protein.

The invention should not be limited to the examples described above.

Example 5 - Expression of Random Sequence Genes in Non-Fusion Vectors

Synthetic genes can be cloned into expression vectors such that the polypeptide products are not fused to vector-derived protein sequences. As in fusion vectors, these non-fusion vectors contain the appropriate transcriptional and translational signals; however, the synthetic genes are linked directly adjacent to the translation initiation signal such that they contain a methionine residue as the amino-terminal amino acid. This single methionine can be removed from COP-1 polypeptides by cyanogen bromide cleavage. COP-1 polypeptides with and without this amino-terminal methionine are tested for biological activity.

Example 6 - Purification of rCOP-1 Polypeptides

The purification of rCOP-1 polypeptides can be accomplished by a number of methods which are well known to those skilled in this art. For example, E. coli cells expressing Protein A/rCOP-1 fusion proteins are grown in a fermenter, collected by centrifugation, and lysed using a dynamill. The extract is centrifuged to remove debris, adjusted to contain 8 M urea, and chromatographed on an S-Sepharose column using a sodium chloride gradient for elution. Fractions containing the fusion protein are dialyzed against a solution of glycerol in phosphate buffered saline; the dialysate is centrifuged to remove contaminating proteins that precipitate during dialysis. The Protein A/rCOP-1 fusion protein is cleaved with cyanogen bromide and the rCOP-1 polypeptide is purified by gel filtration and reverse phase HPLC.

Example 7 - EAE Experiments

rCOP-1 has been tested for efficacy in suppressing experimental allergic encephalomyelitis (EAE). As described above, EAE is a T-cell mediated autoimmune disease that is employed as a model for the human disease multiple sclerosis. EAE experiments are performed essentially as described by Swanborg (Swanborg, R.H. [1988] "Experimental Allergic Encephalomyelitis," In Methods in Enzymology, vol. 162, p. 413, Academic Press, Inc.). For example, the disease is induced in Hartley guinea pigs by a single, subcutaneous injection of 10 μg of guinea pig myelin basic protein in Freund's adjuvant containing 100 μg of Mycobacterium tuberculosis. Onset of disease occurs about 12 to 20 days after induction. The disease is scored on a scale of 0-4: 0 = no disease; 1 = loss of coordination in hind limbs; 2 = paralysis of one or both hind limbs; 3 = paralysis extending to include one or both front limbs, can include incontinence of bladder or bowel; and 4 = extensive paralysis, inability to move. Animals are scored every 2-3 days from the onset of disease, and most animals spontaneously recover from the disease. The treatment protocol consists of intramuscular injections of 500 mg of test material at 1, 6, and 11 days after induction of disease. The dosage, route of administration, and schedule for treatments can be varied. Also, EAE experiments can be performed in other species including rats and mice. Other variations of the experimental protocols and scoring may be used.

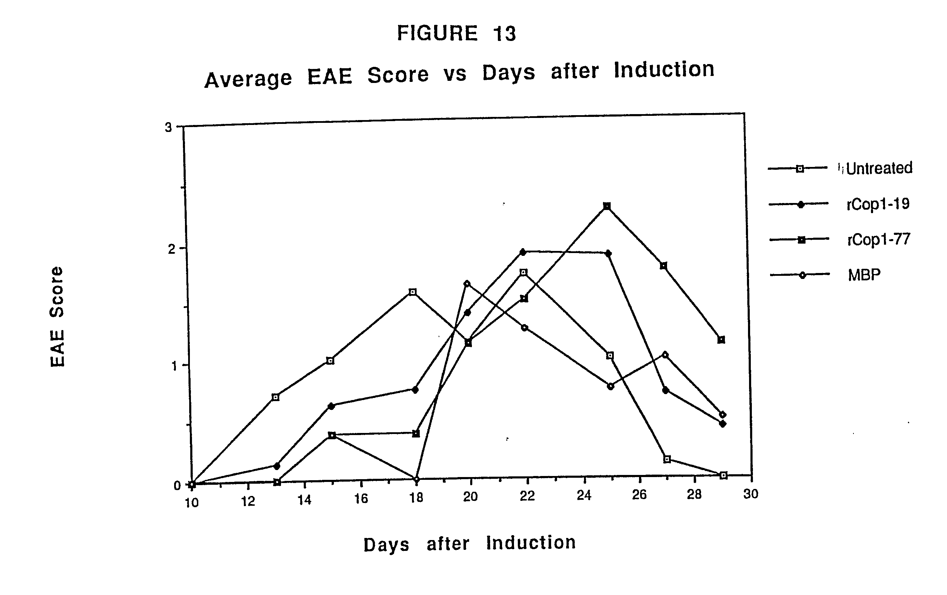

Two rCOP-1 molecules, rCOP-1-77 and rCOP-1-19, have recently been tested in the EAE experiments. The production and purification of these molecules were performed in accordance with the procedures described in Examples 4 and 6. Guinea pigs treated with rCOP-1-77 or rCOP-1-19 were compared to animals treated with myelin basic protein, a positive control, and to an untreated group. The graph in Figure 13 shows the average EAE scores for each treatment group versus days after induction. In Table 1, several aspects of disease, such as incidence, day of onset, maximum severity, and duration, are compared.

Table 1.

| Effects on rCOP-1 and MBP on EAE | | | | |
|---|---|---|---|---|
| Group Duration[2] | Incidence[1] | Day of Onset[2] | Max. Severity[2] | |
| Untreated | 7/7 | 13.5 ± 1.0 (13-15) | 2.3 ± 0.6 (1-3) | 10.8 ± 3.8 (4-15) |
| rCOP-1-19 | 8/8 | 16.8 ± 3.7 (13-25) | 2.8 ± 1.4 (1-4) | 8.6[3] ± 5.2 (2-15) |
| rCOP-1-77 | 7/8 | 17.6 ± 2.8 (15-22) | 2.9 ± 1.0 (1-4) | 9.9 ± 4.5 (2-15) |
| Myelin Basic Protein | 8/8 | 18.1 ± 2.6 (15-20) | 2.3 ± 1.1 (1-4) | 5.3 ± 5.5 (2-15) |

[1] Incidence = Number with disease/number tested.

[2] Values are the mean standard deviation. Ranges are in parentheses.

[3] One animal in this group died.

The results can be summarized as follows: rCOP-1-77 and rCOP-1-19 both delayed the onset of disease using the treatment regimen described here. The rCOP-1 molecules did not affect other measures of disease-incidence, maximum severity, or duration. Myelin basic protein delayed onset and decreased duration of disease but it did significantly alter severity or incidence. One note about this particular experiment is that the maximum severity for the untreated animals (2.3) is unusually low; in a pilot experiment with two guinea pigs, the severity scores were 2 and 4. It may be possible to optimize the effects of rCOP-1 by varying the treatment procedure.

Example 8 - Other Applications for Random Sequence Polymers of Amino Acids

Genes encoding random sequence polypeptides composed of other amino acids can be synthesized using the procedures described herein. Since the properties of the polypeptides are dependent on the amino acid composition and length, additional applications for such polypeptides can be anticipated.

Random sequence polypeptides of predetermined amino acid composition may be useful additives to hair care products. One common type of damage to hair is due to the reduction of disulfide bonds and the subsequent oxidation of cysteine residues to cysteic acid, changes that produce undesirable effects on hair. Random sequence polypeptides may be able to interact with damaged hair and neutralize these effects. Polypeptides of different lengths, amino acid compositions, and sequences will have different physical properties such as charge, solubility, and ability to absorb ultraviolet light. These molecules may confer beneficial effects on damaged hair, such as increasing the strength of the hair or the ease of combing the hair. The effects may vary depending on the physical properties of the polypeptide. For example, a positively charged polypeptide with tryptophan residues may be able to neutralize the negative charge of cysteic acid residues and, in addition, decrease damage caused by exposure to sunlight since the tryptophan will absorb ultraviolet light.

Another potential use for random sequence polypeptides having a predetermined amino acid composition is as supplements for diets deficient in certain amino acids.

**Claims**

1. A method for making a synthetic gene encoding a random polymer of amino acids where said polymer has predetermined amino acid constituents, said method comprising the polymerization of small oligonucleotide duplexes.

2. A method, according to claim 1, wherein the length of said synthetic genes is controlled through the use of adaptors specific for the 5' and 3' ends of the oligonucleotide.

3. A method, according to claim 1, wherein said small oligonucleotide duplexes consist of nucleotides where the number of nucleotides is a multiple of 3.

4. A method, according to claim 3, wherein the size of said small oligonucleotide duplexes is selected from the group consisting of 3-mers, 6-mers, 9-mers, 12-mers, 15-mers, and 18-mers.

5. A method, according to claim 1, wherein the sticky ends of said duplexes are the same for each duplex so that the duplexes can align and ligate in any order.

6. A method, according to claim 1, wherein the relative proportion of amino acid constituents in said polymer is determined by the proportion of oligonucleotide duplexes which are incorporated.

7. A method, according to claim 1, wherein said synthetic gene codes for a polypeptide consisting substantially of alanine, lysine, and either glutamic or aspartic acid.

8. A method, according to claim 1, wherein said synthetic gene codes for a polypeptide consisting substantially of alanine, lysine, either glutamic or aspartic acid, and tyrosine.

9. A method, according to claim 1, wherein said synthetic gene codes for a polypeptide consisting substantially of alanine, lysine, either glutamic or aspartic acid, and either tyrosine or tryptophan.

10. A method, according to claim 1, wherein said polypeptide has a molecular weight between about 5,000 and about 50,000 daltons.

11. A method, according to claim 1, wherein said synthetic gene codes for a polypeptide which is capable of preventing, arresting, or controlling experimental autoimmune encephalomyelitis.

12. A method, according to claim 1, wherein said synthetic gene encodes for a polypeptide which is capable of preventing, arresting, or controlling a demyelinating disorder.

13. A method, according to claim 12, wherein said demyelinating disorder is multiple sclerosis.

14. A method for making polypeptides of random amino acid sequence where said polypeptides have predetermined amino acid constituents, said method comprising the synthesis of genes encoding said random polymers, said synthesis comprising the polymerization of small oligonucleotide duplexes, said method for making polypeptides further comprising the cloning of said synthetic genes into an expression vector and transferring said vector into a bacteria or eukaryotic cell capable of expressing said polypeptide.

15. A method, according to claim 14, wherein said bacteria is an Escherichia coli.

16. A method for making a fusion polypeptide having one portion comprised of all or part of a heterologous polypeptide and a second portion comprised of a random sequence wherein said random sequence portion has predetermined amino acid constituents, and said method comprises the synthesis of genes encoding said random polymers, said synthesis comprising the polymerization of small oligonucleotide duplexes, said method further comprising the cloning of said synthetic genes into an expression vector adjacent to a DNA sequence encoding all or part of said heterologous polypeptide and transferring said vector into a bacteria or eukaryotic cell capable of expressing said fusion polypeptide.

17. A method for synthesizing and identifying a biologically or immunologically active polypeptide, or mixture of polypeptides, said method comprising the following steps:

(a) synthesizing genes encoding random polypeptides by polymerization of small oligonucleotide duplexes;

(b) cloning each of said synthetic genes into a vector and transferring said vector into a host capable of expressing said polypeptides;

(c) growing said hosts under conditions which permit the formation of recombinant colonies which each express one recombinant gene;

(d) testing the polypeptide, or a mixture of the recombinant polypeptides, combined either before or after isolation from said colonies, for evidence of biological and/or immunological activity;

(e) where activity is observed for the mixture of polypeptides, generating smaller subsets of that combination and testing each of these subsets for biological activity; and

(f) repeating step (e) until the active component(s) or a suitable mixture thereof is obtained.

18. A synthetic gene which codes for rCOP-1-19.

19. A synthetic gene which codes for rCOP-1-77.

Figure 1

## Strategy for Synthesizing Random Genes and
## Identifying Recombinant Polypeptides with Biological Activity

mixture of
oligonucleotide
duplexes

ligate

random sequence
synthetic genes

clone in an expression vector

transform into host

recombinant colonies,
each contains one
synthetic gene

identification of biologically active polypeptides

pool colonies (e.g.1000)                          select individual colonies

isolate polypeptides                              isolate the polypeptide

test for biological activity                      test for biological activity

fractionate into smaller pools of
colonies (e.g. 10 pools of 100
colonies)

EP 0 383 620 A2

Figure 2

## Synthesis of Genes Encoding
## Random-Sequence
## Amino Acid Polymers

Oligonucleotides (9-mers)

anneal

Duplexes

ligate

Synthetic Genes

expression

Polypeptides

Ala-Ala-Ala-Lys-Lys-Lys-Ala-Ala-Ala-Lys-Lys-Lys-Ala-Ala-Ala

Ala-Ala-Ala-Ala-Ala-Ala-Lys-Lys-Lys-Lys-Lys-Lys-Lys-Lys-Lys

Lys-Lys-Lys-Ala-Ala-Ala-Ala-Ala-Ala-Lys-Lys-Lys-Ala-Ala-Ala

Figure 3

# All Possible 3 Amino Acid Combinations and Their Percent Occurence in Cop 1

| | | | | | | | |
|---|---|---|---|---|---|---|---|
| AAA | 7.872 | AAE | 2.624 | AAK | 6.560 | AAY | 1.312 |
| AEA | 2.624 | AEE | 0.875 | AEK | 2.187 | AEY | 0.437 |
| AKA | 6.560 | AKE | 2.187 | AKK | 5.466 | AKY | 1.093 |
| AYA | 1.312 | AYE | 0.437 | AYK | 1.093 | AYY | 0.219 |
| EAA | 2.624 | EAE | 0.875 | EAK | 2.187 | EAY | 0.437 |
| EEA | 0.875 | EEE | 0.292 | EEK | 0.729 | EEY | 0.146 |
| EKA | 2.187 | EKE | 0.729 | EKK | 1.822 | EKY | 0.364 |
| EYA | 0.437 | EYE | 0.146 | EYK | 0.364 | EYY | 0.073 |
| KAA | 6.560 | KAE | 2.187 | KAK | 5.466 | KAY | 1.093 |
| KEA | 2.187 | KEE | 0.729 | KEK | 1.822 | KEY | 0.364 |
| KKA | 5.466 | KKE | 1.822 | KKK | 4.555 | KKY | 0.911 |
| KYA | 1.093 | KYE | 0.364 | KYK | 0.911 | KYY | 0.182 |
| YAA | 1.312 | YAE | 0.437 | YAK | 1.093 | YAY | 0.219 |
| YEA | 0.437 | YEE | 0.146 | YEK | 0.364 | YEY | 0.073 |
| YKA | 1.093 | YKE | 0.364 | YKK | 0.911 | YKY | 0.182 |
| YYA | 0.219 | YYE | 0.073 | YYK | 0.182 | YYY | 0.036 |

| |
|---|
| A=Alanine |
| E=Glutamic Acid |
| K=Lysine |
| Y=Tyrosine |

Figure 4

## 9-Nucleotide Duplexes and Adaptors
## for Cop 1 Gene Synthesis

**Examples of 9-Nucleotide Duplexes for Cop 1 Genes:**

Coding       (5' end )   A A G A A G G C A      G A A G C A G A A     (3' end)

Noncoding    (3' end ) T T T C T T C C G    T C T T C G T C T   (5' end)

Amino acids          K   K · A       E    A    E

**Adaptors:**

For the 5' end

GATC ———— A
↑
(BamH I)          P

For the 3' end

P
———AGCT
T ————        (Sac I)
OH

Figure 5

## Construction and Cloning Synthetic COP 1 Genes

Figure 6

## Sequence Analysis of a Synthetic Gene

amino acids:   Y    K    K    E    A    E    K    A    K

nucleotides:  T A C A A G A A A G A A G C A G A A A A G G C T A A A

amino acids:   Y    K    K    Y    K    K    K    K    A

nucleotides:  T A C A A G A A A T A C A A G A A A A A G A A G G C A

amino acids:   E    A    E    K    K    A    E    A    E

nucleotides:  G A A G C A G A A A A G A A G G C A G A A G C A G A A

amino acids:   K    K    A    E    A    E    E    A    E

nucleotides:  A A G A A G G C A G A A G C A G A A G A A G C A G A A

amino acids:   K    K    A    K    A    K    E    A    E

nucleotides:  A A G A A G G C A A A G G C T A A A G A A G C A G A A

amino acids:   K    K    A    K    K    A    K    A    K

nucleotides:  A A G A A G G C A A A G A A G G C A A A G G C T A A A

The lines in the nucleotide sequence mark the junctions
between 9-mer duplexes.

Amino Acids:
 A= alanine
 K=lysine
 E=glutamic acid
 Y=tyrosine

Figure 7

Figure 8

## Variations of Random-Gene Synthesis using Small Duplexes

### One Nucleotide Extension:

3-mers    GCA               GAA
           TCG               TCT

6-mers    GCAAAA        GAAGCA
           TCGTTT         TCTTCG

12-mers     ·    GCAGAAGCAAAA
                 TCGTCTTCGTTT

### Three Nucleotide Extensions. 12-mer Duplexes:

duplex                XXXXXXXXXGCA
                     CGTXXXXXXXXX

ligate

gene        XXXXXXXXXGCAXXXXXXXXXGCAXXXXXXXXXGCA
          CGTXXXXXXXXXCGTXXXXXXXXXCGTXXXXXXXXX

polypeptide    Ala aa aa aa Ala aa aa aa Ala aa aa aaa Ala

xxx= any codon
aa= amino acid specified
    by codon xxx
Ala= alanine

Figure 9

**Synthesis of Single-Stranded DNA Encoding
Random Sequence Amino Acid Polymers**

## 3 Nucleotide Segments for COP I

| | Ala | Glu | Lys | Tyr |
|---|---|---|---|---|
| | 5'GCA3' | 5'GAA3' | 5'AAA3' | 5'TAC3' |
| ratio: | 6 | 2 | 5 | 1 |

Polymerize
or A. Solution Chemistry
B. DNA Synthesizer

Single-Stranded DNA        5' ———————————————— 3'

Enzymatic Synthesis
of the Second Strand

Double-Stranded DNA        5' ———————————————— 3'
3' — — — — — — — — 5'

Clone into
Expression Vector

Plasmid
Rev 2.1

Figure 10

**Phosphoramidite Trinucleotide for Random
Gene Synthesis Using a DNA Synthesizer**

DMTr
|
O
|
$CH_2$   O   $B_1$

**Phosphoramidite
Trinucleotide**

O
|
$O=P—O$
|
O
|
$CH_2$   O   $B_2$

O
|
$O=P—O$
|
O
|
$CH_2$   O   $B_3$

O
|
P

$CN-CH_2-CH_2O$   $N—CH(CH_3)_2$
|
$CH(CH_3)_2$

**Phosphoramidite
Mononucleotide**

DMTr
|
O
|
$CH_2$   O   B

O
|
P

$CH_3O$   $N—CH(CH_3)_2$
|
$CH(CH_3)_2$

EP 0 383 620 A2

# FIGURE 11

## rCOP-1-77

```
GCATACAAGAAAGAAGCAGAATACAAGAAATACAAGAAATACAAGAAAGAAGCAGAAGAA
-----+---------+---------+---------+---------+---------+----
CGTATGTTCTTTCTTCGTCTTATGTTCTTTATGTTCTTTATGTTCTTTCTTCGTCTTCTT

 A   Y   K   K   E   A   E   Y   K   K   Y   K   K   Y   K   K   E   A   E   E   -

GCAGAATACAAGAAAAAGGCTAAAGAAGCAGAAAAGGCAAAAAAGGCTAAATACAAGAAA
-----+---------+---------+---------+---------+---------+----
CGTCTTATGTTCTTTTTCCGATTTCTTCGTCTTTTCCGTTTTTTCCGATTTATGTTCTTT

 A   E   Y   K   K   K   A   K   E   A   E   K   A   K   K   A   K   Y   K   K   -

TACAAGAAAGAAGCAGAAGCTGCTAAAGCTGCTAAAGCTGCTGCAGCTGCTGCATACAAG
-----+---------+---------+---------+---------+---------+----
ATGTTCTTTCTTCGTCTTCGACGATTTCGACGATTTCGACGACGTCGACGACGTATGTTC

 Y   K   K   E   A   E   A   A   K   A   A   K   A   A   A   A   A   A   Y   K   -

AAAGAAGCAGAAGCTGCTGCAGAAGCAGAAAAAGGCTAAATACAAGAAAAAGGCTAAAGAA
-----+---------+---------+---------+---------+---------+----
TTTCTTCGTCTTCGACGACGTCTTCGTCTTTTCCGATTTATGTTCTTTTTCCGATTTCTT

 K   E   A   E   A   A   A   A   E   A   E   K   A   K   Y   K   K   K   A   K   E   -

GCAGAATACAAGAAAAAGGCTAAAGCTGCTGCAGAAGCAGAATACAAGAAAGAAGCAGAA
-----+---------+---------+---------+---------+---------+----
CGTCTTATGTTCTTTTTCCGATTTCGACGACGTCTTCGTCTTATGTTCTTTCTTCGTCTT

 A   E   Y   K   K   K   A   K   A   A   A   E   A   E   Y   K   K   E   A   E   -

GAAGCAGAATACAAGAAATACAAGAAAAAGGCTAAAAAGGCTAAATACAAGAAAAAGGCT
-----+---------+---------+---------+---------+---------+----
CTTCGTCTTATGTTCTTTATGTTCTTTTTCCGATTTTTCCGATTTATGTTCTTTTTCCGA

 E   A   E   Y   K   K   Y   K   K   K   A   K   K   A   K   Y   K   K   K   A   -

AAAGAAGCAGAAAAGGCTAAAGCTGCTGCAGAAGCAGAAAAAGGCTAAAGAAGCAGAATAC
-----+---------+---------+---------+---------+---------+----
TTTCTTCGTCTTTTTCCGATTTCGACGACGTCTTCGTCTTTTCCGATTTCTTCGTCTTATG

 K   E   A   E   K   A   K   A   A   A   E   A   E   K   A   K   E   A   E   Y   -

AAGAAATACAAGAAAGAAGCAGAAAAGGCTAAAGAAGCAGAATAA
-----+---------+---------+---------+---------
TTCTTTATGTTCTTTCTTCGTCTTTTTCCGATTTCTTCGTCTTATT

 K   K   Y   K   K   E   A   E   K   A   K   E   A   E   *   -
```

# FIGURE 12
## rCOP-1-19

```
GCAAAGGCTGCAGAGAAAGCAAAGGCTGCAAAGAAGGCATACGAAGCAGAGAAAGCAAAG
---------+---------+---------+---------+---------+---------+
CGTTTCCGACGTCTCTTTCGTTTCCGACGTTTCTTCCGTATGCTTCGTCTCTTTCGTTTC
 A  K  A  A  E  K  A  K  A  A  K  K  A  Y  E  A  E  K  A  K   -

GCTAAATACGAAGCAAAGAAGGCAGAGAAAGCAGAGAAAGCAGAGAAAGCAGCTGCTGAA
---------+---------+---------+---------+---------+---------+
CGATTTATGCTTCGTTTCTTCCGTCTCTTTCGTCTCTTTCGTCTCTTTCGTCGACGACTT
 A  K  Y  E  A  K  K  A  E  K  A  E  K  A  E  K  A  A  A  E   -

AAGAAGGCAAAGGAAGCAAAGAAGGCAGAGAAAGCAAAGGAAGCAGAGAAAGCAAAGGAA
---------+---------+---------+---------+---------+---------+
TTCTTCCGTTTCCTTCGTTTCTTCCGTCTCTTTCGTTTCCTTCGTCTCTTTCGTTTCCTT
 K  K  A  K  E  A  K  K  A  E  K  A  K  E  A  E  K  A  K  E   -

GCAGAGAAAGCAAAGGAAGCAAAGAAGGCAAAGGCTGCAGAGAAAGCAGAGAAAGCAAAG
---------+---------+---------+---------+---------+---------+
CGTCTCTTTCGTTTCCTTCGTTTCTTCCGTTTCCGACGTCTCTTTCGTCTCTTTCGTTTC
 A  E  K  A  K  E  A  K  K  A  K  A  A  E  K  A  E  K  A  K   -

AAGGCAGAGAAAGCAAAGGCTGCAGAGAAAGCAAAGGCTGCATACAAGAAATACAAGAAA
---------+---------+---------+---------+---------+---------+
TTCCGTCTCTTTCGTTTCCGACGTCTCTTTCGTTTCCGACGTATGTTCTTTATGTTCTTT
 K  A  E  K  A  K  A  A  E  K  A  K  A  A  Y  K  K  Y  K  K   -

GCGAAAGCAAAGGCTGCATAA
---------+---------+-
CGCTTTCGTTTCCGACGTATT
 A  K  A  K  A  A  *   -
```

FIGURE 13

Average EAE Score vs Days after Induction

Days after Induction

EP 0 383 620 A2